# EUROPÄISCHE PATENTANMELDUNG

(11) **EP 0 604 709 A2**
(43) Veröffentlichungstag der Anmeldung: **06.07.1994**
(21) Anmeldenummer: 93114280.6
(22) Anmeldetag: 06.09.1993
(51) Int. Cl.: C09J 133/06, C09J 4/00, C09J 4/06

(54) **Haftschmelzkleber, Verfahren zu ihrer Herstellung und ihre Verwendung**

(30) Priorität: 28.09.1992 DE 4232472
(71) Anmelder: LOHMANN GmbH & Co. KG, D-56513 Neuwied (DE)
(72) Erfinder: Czech, Zbigniew, Dr. Dipl.-Chem., D-56075 Koblenz (DE)
(74) Vertreter: Neidl-Stippler, Cornelia, Dr.

(57) **Zusammenfassung**

Die Erfindung betrifft Haftschmelzkleber aus strahlungsvernetzbaren Haftschmelzkleberzusammensetzungen, Verfahren zu ihrer Herstellung, wobei eine Mischung zwischen den Haftschmelzkleberkomponenten und dem strahlungsinitiierbaren Vernetzer hergestellt wird, der Haftschmelzkleber in geschmolzenem Zustand auf Verarbeitungstemperatur auf ein Substrat aufgebracht wird und der strahlungsvernetzbare Vernetzer durch Bestrahlung zur Vernetzungsreaktion gebracht wird, sowie die Verwendung des Haftschmelzklebers für Pflaster, transdermale therapeutische Systeme, Klebebänder, Etiketten, Zierleisten usw..

## Beschreibung

Die Erfindung betrifft Haftschmelzkleber, Verfahren zu ihrer Herstellung sowie ihre Verwendung.

Radikalisch nachvernetzbare, insbesondere nachvernetzbare Haftkleber auf Acrylatbasis sind bereits aus der DE-PS 37 40 324 bekannt geworden. Dabei handelt es sich um Haftkleber, wie sie zur Beschichtung von Klebebändern, Etiketten, etc. dienen, bei denen es wesentlich ist, daß sie leicht ver-arbeitet, d.h. auf das jeweilige Substrat aufgetragen werden können. Bei der Verarbeitung ist daher eine niedere Viskosität erwünscht, die aber mit einer geringen Kohäsion einhergeht, während beim daraus hergestellten haftklebenden Substrat, wie einem Klebeband, Pflaster, transdermalen therapeutischen System oder dgl. eine hohe Kohäsion des Klebers mit guter Anfaßklebrigkeit verbunden sein soll. Dabei ist es insbesondere erwünscht, eine hohe Kohäsion des Klebers mit möglichst hoher Adhäsion und hoher Klebekraft zur erreichen. Um die Kohäsion von Klebstoffen zu verbessern, wurde bereits vorgeschlagen, sogenannte strahlungsinitiierbare Vernetzer einzusetzen.

Viele derartige Vernetzer sind bekannt und bspw. von R. Holman und P. Oldring; "UV- and EB-curing formulations for printing inks, coatings and paints", 1988, published by SITA-Technology, 203 Gardena House, Brookhill Road, London, S.W. 18, Great Britain,beschrieben.

Als Grundpolymere können bspw. Polyamide, Polyester, Polycaprolactame, Polycaprolacton, Ethylen-Vinylacetat-Copolymerer (EVA), Ethylen-Ethylacrylat-Copolymere (EEA), Polyvinylether, Polyacrylatester, Polyvinylacetale, Polyvinylacetate, Styrol-Butadien-Blockpolymere, Isopren-Blockpolymerer, Polyurethane, Ethylcellulose, Cellulosecetat-Butyrat; Synthesekautschuke (z.B. Neopren-Kautschuk), Polyisobutylen, Butylgummi, Acrylnitril-Butadien-Mischpolymerisate, Epoxidharze, Melaminharze, Phenol-formaldehyd-Harze und Resorcin-Formaldehyd-Harz vernendet werden, wobei auch unter anderem modifizierende Harze eingesetzt werden können.

Eine Aufstellung geeigneter Haftschmelzkleber, auch niedrigschmelzender, findet sich in der DE-PS P 37 43 947, der DE-PS 37 43 945 und der DE-PS 37 43 946, auf deren Offenbarung zur Vermeidung von Wiederholung in vollem Umfang bezug genommen wird.

Haftklebstoffsysteme mit strahleninitiierten Vernetzern, die aus Lösung aufgetragen werden, insbesondere solche auf Acrylatbasis sind bereits bekannt, so aus der DE-PS 1719169 der PG Industries Inc., der DE-PS 18 12 887 (National Starch and Chem. Corp.), der DE-PS 19 61 615 (JOHNSON and JOHNSON) der DE-OS 20 45 985 (The B.F. Goodrich Corp.), der DE-PS 26 56 521 (ETAFIN Corp. S.A.).

Diese nachvernetzbaren Haftkleberzusammensetzungen sind für die Praxis insofern mit gravierenden Nachteilen bei der Herstellung der klebenden Substrate behaftet, da bei der bekannten Herstellung lösemittelhaltige Haftklebstoffe in Mischung mit Vernetzern aufgebracht, getrocknet und dann radikalisch nachvernetzt wurden. Die Verwendung von Lösemitteln ist unter anderem aus den nachfolgenden Gründen problematisch geworden:
Die Kosten für Lösemittel steigen ständig, insbesondere auch für reine Lösemittel, die praktisch rückstandsfrei verdampfen, wie dies insbesondere für pharmazeutische Anwendungen, bei denen eine Irritation der Haut durch teilweise toxische oder zumindest als Reizstoffe oder auch Allergene wirkende Lösemittelreste in der Haftklebstoffschicht vermieden werden soll.

Die Arbeitsplatzschutzvorschriften für die Verwendung von Lösemitteln und der daraus folgende Aufwand für Absaugstrecken werden ständig verschärft.

Schließlich werden Lösemittel, insbesondere halogenierte Lösemittel oder auch Kohlenwasserstoffe wegen der durch diese verursachten Umweltbelastung, einschließlich eines negativen Effekts auf die Ozonschicht, strengen amtlichen Bestimmungen unterworfen, wobei diese Anforderungen ständig steigen.

Sowohl aus Kosten- als auch aus Umweltschutzgründen wird daher angestrebt, die Verwendung von Lösemitteln, insbesondere bei der Auftragung von Klebestoffen möglichst zu umgehen.

Es ist bereits vorgeschlagen worden, für diesen Zweck Haftschmelzkleber, wie sie bspw. aus der Buchbindetechnik bekannt sind, einzusetzen.

Diese bekannten Haftschmelzkleber hatten allerdings bisher den Nachteil, daß sie häufig auch im geschmolzenen Zustand keine ausreichend geringe Viskosität und Fließfähigkeit aufwiesen. Durch Einsatz höherer Verarbeitungstemperaturen, die zu einer besseren Verflüssigung der Kleber führen kann - sofern die Kleberzusammensetzung höhere Temperaturen verträgt, wird allerdings auch eine höhere Temperaturbelastung des zu beschichtenden Substrats erhalten. Hohe Temperaturen eignen sich insbesondere nicht für Substrate aus Kunststoffen oder auch Haftkleberzusammensetzungen mit flüchtigen Bestandteilen.

Schließlich führt die Verwendung hoher Temperaturen auch zu einem höheren Energieverbrauch, was bei der Herstellung großer Mengen vermieden werden soll.

Es ist demzufolge Aufgabe der Erfindung, Haftkleberzusammensetzungen zu finden, die leicht, d.h. mit geringer Viskosität zu verarbeiten sind, deren Eigenschaften im aufgetragenen Zustand aber eine hohe Kohäsion und Adhäsion zeigen.

Gleichzeitig soll der Einsatz von Lösemitteln eingeschränkt werden.

Die Aufgabe wird erfindungsgemäß gelöst durch nachvernetzbare Haftschmelzkleber.

Vorteilhafte Weiterbildungen der Erfindung ergeben sich aus den Unteransprüchen.

Bevorzugt handelt es sich dabei um strahlennachvernetzbare Haftschmelzkleber.

Eine besonders bevorzugte Ausführungsform dieser nachvernetzbaren Haftschmelzkleber sind nachvernetzbare Haftkleber auf Acrylatbasis, mit einem Gehalt von 0,01 bis 15 Gew.%, bevorzugt 0,1 bis 10 Gew.% einer Verbindung der allgemeinen Formel: 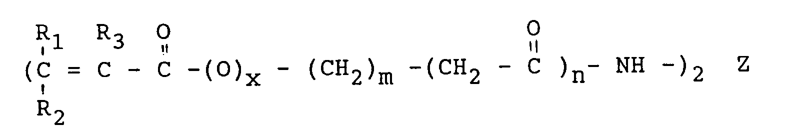
wobei R₁, R₂ = H oder F, Cl, Br oder J ; x = 0 oder 1; n = 0,1; Z = 2,4-Toluylrest, 2,6-Toluylrest oder 1,5-(1-Methylen-1,3,3-trimethyl)-cyclohexylenrest; R₃ = H, CN, Halogen oder CH₃; m = 0 - 12 sein können, als Kreuzvernetzer.

Bevorzugte Haftschmelzkleber auf Acrylatbasis sind ausgewählt aus der Gruppe bestehend aus: Methylacrylat, Ethylacrylat, Butylacrylat, Hexylacrylat, Heptylacrylat, 2-Ethylhexyl-acrylat, 2-Methylheptylacrylat, Isooctylacrylat, n-Nonyl-acrylat, Isononylacrylat, Decylacrylat und/oder Dodecyl(meth)acrylat.

Die Erfindung bezieht sich ferner auf die Verwendung der nachvernetzbaren Haftschmelzkleber für Pflaster, transdermale therapeutische Systeme, Klebebänder u. dgl., wobei darauf hinzuweisen ist, daß diese Haftschmelzkleber selbstverständlich auch Wirkstoffe enthalten können, wie sie bspw. aus der DE-PS 37 43 946 bekannt sind.

Ferner betrifft die Erfindung auch ein Verfahren zur Herstellung von Haftschmelzklebern, wobei man eine Mischung zwischen den Haftschmelzkleberkomponenten und dem Vernetzer sowie ggf. weiteren Bestandteilen, wie Wirkstoffen, Tackifiern, Füllstoffen, Alterungsschutzmitteln etc. herstellt und schmilzt, diese Haftschmelzklebermischung im geschmol-zenen Zustand auf ein Substrat aufbringt und anschließend den/die Vernetzer durch durch Bestrahlung zur Vernetzungs-reaktion bringt und dadurch die Viskosität und die Kohäsion der aufgetragenen Mischung erhöht und derart den Haft-kleberauftrag fertigstellt.

Es ist besonders vorteilhaft, wenn der Haftschmelzkleber ein Kleber auf Acrylatbasis ist.

Eine besonders vorteilhafte Verwendung des erfindugsgemäßen nachvernetzbaren Haftschmelzklebers ist für Pflaster, transdermale Systeme, Klebebänder u. dgl.

Ein erfindungsgemäßes Verfahren zur Herstellung des Haftschmelzklebers weist auf, daß man eine Mischung zwischen den Haftschmelzkleberkomponenten und dem Vernetzer sowie ggf. weiteren Komponenten, wie Wirkstoffen, Weichmachern, Füllstoffen od. dgl. herstellt, den Haftschmelzkleber im geschmolzenen Zustand auf Verarbeitungstemperatur auf ein Substrat aufbringt und die strahlungsinitiierbaren Vernetzer durch Bestrahlung zur Vernetzungsreaktion bringt.

Dabei können unterschiedliche Bestrahlungsformen eingesetzt werden, wie UV-Bestrahlung, Elektronenstrahlung od. dgl.

Dadurch, daß nun erfindungsgemäß neue nachvernetzbare Haftklebermischungen gefunden wurden, ist es möglich, Haftkleber ohne Lösemittel aufzubringen und nachzuvernetzen, wodurch eine erhöhte Kohäsion des Haftklebers bei guter Adhäsion und einfacher Verarbeitbarkeit resultiert.

Nachfolgend soll die Erfindung anhand von Beispielen sowie der begleitenden Zeichnung näher erläutert werden. Dabei zeigt:
- Fig. 1: eine Darstellung der Haltedauer einer Klebung mit dem vernetzten Haftschmelzkleber des Beispiels 1.1 in Abhängigkeit von der Belastungsdauer

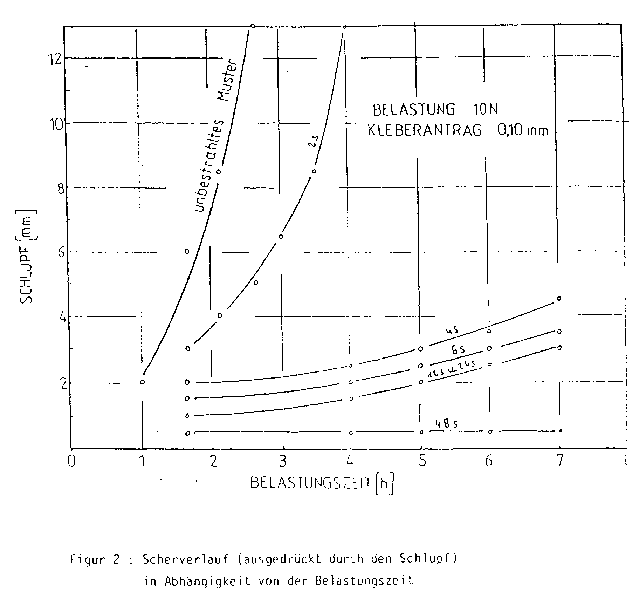

- Fig. 2: eine Auftragung des Schlupfes einer Klebung mit dem Haftkleber des Beispiels 2 in Abhängigkeit von der Belastungsdauer; und
- Fig. 3: eine Auftragung der Klebekraft bei Raumtemperatur in Abhängigkeit von der Bestrahlungszeit.

### Beispiel 1

### Herstellung des Vernetzers

### 1.1. Reaktion von Isophorondiisocyanat mit Acrylsäure zur Herstellung eines an sich bekannten N-substituierten polyfunktionellen ungesättigten Carbonsäureamids.

100 g Isophorondiisocyanat und 65 g Acrylsäure sowie 150 g Ethylacetat wurden in einen Dreihalskolben gebracht und 1 Stunde am Rückfluß gekocht. Danach wurde abgekühlt und das Lösemittel abgezogen. Man erhielt eine weiße Substanz mit einem Zersetzungspunkt von ca 350oC.

### 1.2. Verarbeitung eines strahlungsvernetzbaren Acrylatschmelzklebers

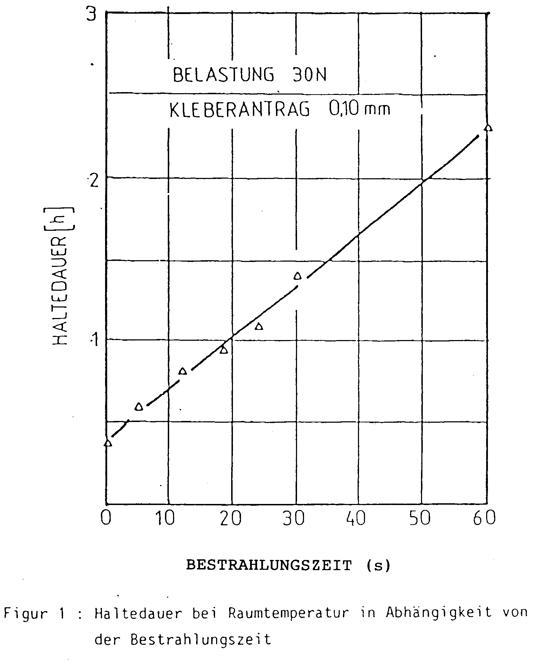

Über etwa zwei Stunden wurde ein Gemisch von 200 g einer Haft-schmelzkleberzusammensetzung auf Butylacrylatbasis, 6 g Pho-toinitiator Iracure 184 und 3g der eingangs hergestellten Verbindung gemischt, polymerisiert und gerührt. Die Mischung wurde auf eine Verarbeitungstemperatur von 110 ^{o}C gebracht und eine viskose, streichfähige klebrige Masse mit geringer Viskosität und geringer Kohäsion erhalten. Diese Masse wurde auf eine Polyesterfolie mit einer Stärke von 0,5 mm bei einer Temperatur von 110^{o}C aufgetragen. Das derart beschichtete Sub-strat wurde unter einer UV-Bestrahlungsstrecke, in der zwei 500 W-UV-Lampen brannten, zur Vernetzungsreaktion gebracht. Nach der Bestrahlung zeigte die Haftkleberschicht eine erheblich verbesserte Kohäsion, wie aus Fig.1 ersichtlich.

### Beispiel 2

### 2.1. Reaktion von Isophorondiisocyanat mit β-Carboxylethylacrylat zur Herstellung eines an sich beannten N-substi-tuierten polyfunktionellen ungesättigten Carbonsäureamids.

100 g Isophorondiisocyanat und 195 g β-carboxyethylacrylat sowie 300 g Ethylacetat wurden in einen Dreihalskolben gebracht und eine Stunde am Rückfluß gekocht. Dnach wurde abgekühlt und das Lösemittel verdampft. Man erhielt eine weiße Substanz mit einem Zersetzungspunkt von ca. 350^{o}C

### 2.2. Verarbeitung des Acrylatschmelzklebers

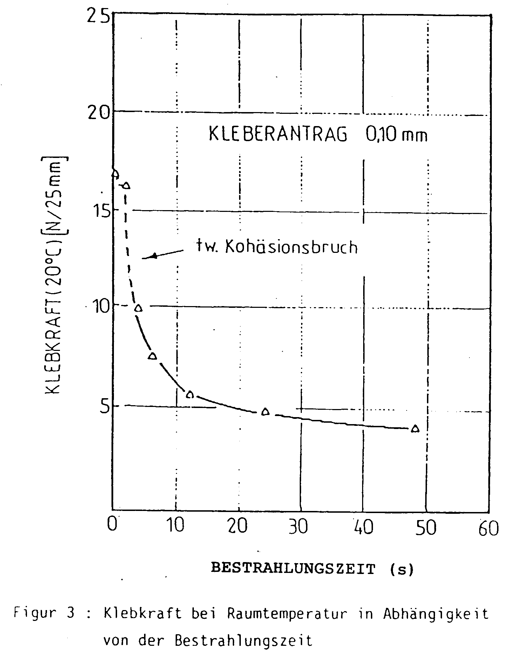

Über ca 2 Std. wurden ein Gemisch von 200 g Haftschmelz-kleberzusammensetzung auf 2-Ethylhexylacrylatbasis und 6 g Photoinitiator Irgacure 184 und 10 g der unter 2.1. herge-stellten Verbindung vermischt, polymerisiert und gerührt. Die Mischung wurde auf eine Verarbeitungstemperatur von 110^{o}C gebracht und eine viskose, streichfähige klebrige Masse mit geringer Viskosität und geringer Kohäsion erhalten. Diese Masse wurde auf eine Polyurethanschaumfolie mit einer Stärke von 1 cm. aufgetragen. Das derart beschichtete Substrat wurde unter einer UV-Bestrahlungsstrecke, in der zwei 500 W-UV-Lampen brannten, zur Vernetzungsreaktion gebracht. Nach der Bestrahlung zeigte die Haftkleberschicht eine erhelblich verbesserte Kohäsion, wie aus Fig.3 ersichtlich.

### Beispiel 3

### 3.1. Reaktion von Isophorondiisocyynat mit Methacrylsäure zur Herstellung eines an sich bekannten N-substituierten polyfunktionellen ungesättigten Carbonsäureamids.

80 g Isophorondiisocyanat und 62 g Methacrylsäure sowie 200 g Acetont wurden in einen Dreihalskolben gebracht und 1 Stunde am Rückfluß gekocht. Danach wurde abgekühlt und das Lösemittel abgezogen. Man erhielt eine weiße Substanz mit einem Zersetzungspunkt von ca 350^{o}C.

### 3.2. Verarbeitung eines Methacrylatschmelzklebers

Über etwa zwei Stunden wurden ein Gemisch von 200 g Haftschmelzkleberzusammensetzung auf Isooctylacrylatbasis, 6 g Photoinitiator Irgacure 184 und 5 g der in 3.1. hergestellten Verbindung gemischt, polymerisiert und gerührt. Die Mischung wurde auf eine Verarbeitungstemperatur von 120^{o}C gebracht und eine viskose, streichfähige klebrige Masse mit geringer Viskosität und geringer Kohäsion erhalten. Diese Masse wurde auf eine Polyesterfolie mit einer Stärke von 50 um aufgetragen. Das derart beschichtete Substrat wurde unter einer UV-Bestrahlungsstrecke, in der zwei 500 W-UV-Lampen brannten, zur Vernetzungsreaktion gebracht. Nach der Bestrahlung zeigte die Haftkleberschicht eine erheblich ver-besserte Kohäsion, wie aus Fig.3 ersichtlich.

### Beispiel 4

### Herstellung des Vernetzers

### 4.1. Reaktion von 2,4-Toluylendiisocyanat mit Methacrylsäure zur Herstellung eines an sich bekannten N-substituierten polyfunktionellen ungesättigten Carbonsäureamids.

100 g 2,4-Toluylendiisocyanat und 100 g Methacrylsäure sowie 200 g Ethylacetat wurden in einen Dreihalskolben gebracht und 1 Stunde am Rückfluß gekocht. Danach wurde abgekühlt und das Lösemittel abgezogen. Man erhielt eine weiße Substanz mit einem Zersetzungspunkt von ca 310^{o}C.

### 4.2. Verarbeitung des Acrylatschmelzklbers

Über etwa zwei Stunden wurden ein Gemisch von 200 g Haftschmelzkleberzusammensetzung auf Butylacrylatbasis, 6 g Photoinitiator Irgacure 184 und 4 g N,N'-Methyacryloyl-2,6-diaminotoluol gemischt, polymerisiert und gerührt. Die Mischung wurde auf eine Verarbeitungstemperatur von 120^{o}C gebracht und eine viskose, streichfähige klebrige Masse mit geringer Viskosität und geringer Kohäsion erhalten. Diese Masse wurde auf eine Polyesterfolie mit einer Stärke von 50um aufgetragen. Das derart beschichtete Substrat wurde unter einer UV-Bestrahlungsstrecke, in der zwei 500 W-UV-Lampen brannten, zur Vernetzungsreaktion gebracht. Nach der Bestrahlung zeigte die Haftkleberschicht eine erheblich verbesserte Kohäsion, wie aus Fig.3 ersichtlich.

### Beispiel 5

### Herstellung des Vernetzers

### 5.1. Reaktion von 2,6-Toluylendiisocyanat mit β-Carboxyethylacrylat zur Herstellung eines an sich bekannten N-substituierten polyfunktionellen ungesättigten Carbonsäureamids.

100 g 2,6-Toluylendiisocyanat und 200 g Carboxylethylacrylat sowie 400 g Ethylacetat wurden in einen Dreihalskolben gebracht und 1 Stunde am Rückfluß gekocht. Danach wurde abgekühlt und das Lösemittel abgezogen. Man erhielt eine weiße Substanz mit einem Zersetzungspunkt von ca 270^{o}C.

### 5.2. Verarbeitung des Acrylathaftschmelzklebers

Über etwa zwei Stunden wurden ein Gemisch von einer Haftschmelzkleberzusammensetzung auf Isononylacrylatbasis, 6 g Photoinitiator Irgacure 184 und 6 g der unter 5.1. her-gestellten Verbindung gemischt, polymerisiert und gerührt. Die Mischung wurde auf eine Verarbeitungstemperatur von 130 ^{o}C gebracht und eine viskose, streichfähige klebrige Masse mit geringer Viskosität und geringer Kohäsion erhalten. Diese Masse wurde auf eine Polyesterfolie mit einer Stärke von 50 um aufgetragen. Das derart beschichtete Substrat wurde unter einer UV-Bestrahlungsstrecke, in der zwei 500 W-UV-Lampen brannten, zur Vernetzungsreaktion gebracht. Nach der Bestrahlung zeigte die Haftkleberschicht eine erheblich verbesserte Kohäsion, wie aus Fig.3 ersichtlich.

### Beispiel 6

### 6.1 Herstellung des Vernetzers

### Reaktion von Isophorondiisocyanat mit Trichloracrylsäure zur Herstellung eines an sich bekannten N-substituierten polyfunktionellen ungesättigten halogenierten Carbonsäureamids.

150 g Isophorondiisocyanat und 237 g Trichlor-Acrylsäure sowie 1 Liter Ethylacetat wurden in einen Dreihalskolben gebracht und 3 Stunden am Rückfluß gekocht. Danach wurd abgekühlt und das Lösemittel abgezogen. Man erhielt eine weiße Substanz mit einem Zersetzungspunkt von ca 280^{o}C.

### 6.2. Verarbeitung des nachvernetzbaren Haftschmelzschmelzklebers auf Acrylatbasis

Über etwa zwei Stunden wurden ein Gemisch 200 g einer Haftschmelzkleberzusammensetzung auf Butylacrylatbasis, 6 g Photoinitiator Irgacure 184 und 3 g N,N'-Trichloracryloyl-1,5-(1,5-(1-Methylen-1,3,3-trimethyl)cyclohexyl gemischt, polymerisiert und gerührt. Die Mischung wurde auf eine Verarbeitungstemperatur von 140^{o}C gebracht und eine viskose, streichfähige klebrige Masse mit geringer Viskosität und geringer Kohäsion erhalten. Diese Masse wurde auf eine Polyesterfolie mit einer Stärke von 50um aufgetragen. Das derart beschichtete Substrat wurde unter einer UV-Bestrahlungsstrecke, in der zwei 500 W-UV-Lampen brannten, zur Vernetzungsreaktion gebracht. Nach der Bestrahlung zeigte die Haftkleberschicht eine erheblich verbesserte Kohäsion, wie aus Fig.3 ersichtlich.

### Beispiel 7

### Herstellung des Vernetzers

### 7.1. Reaktion von Isophorondiisocyanat mit Dibromacrylsäure zur Herstellung eines an sich beannten N-substituierten polyfunktionellen ungesättigten Carbonsäureamids.

100 g Isophorondiisocyanat und 65 g Dibromacrylsäure sowie 150 g Ethylacetat wurden in einen Dreihalskolben gebracht und 1 Stunde am Rückfluß gekocht. Danach wurd abgekühlt und das Lösemittel abgezogen. Man erhielt eine weiße Substanz mit einem Zersetzungspunkt von ca 350^{o}C.

### 7.2. Verarbeitung des Acrylatschmelzklebers

Über etwa zwei Stunden wurden ein Gemisch von 200g einer Haftschmelzkleberzusammensetzung auf Isooctylacrylatbasis, 6 g Photoinitiator Irgacure 184 und 2 g des unter 7.1. hergestellten Vernetzers gemischt, polymerisiert und gerührt. Die Mischung wurde auf eine Verarbeitungstemperatur von 110^{o}C gebracht und eine flüssig-viskose, streichfähige klebrige Masse mit geringer Viskosität und geringer Kohäsion erhalten. Diese Masse wurde auf eine Polyesterfolie mit einer Stärke von 50um aufgetragen. Das derart beschichtete Substrat wurde unter einer UV-Bestrahlungsstrecke, in der zwei 500 W-UV-Lampen brannten, zur Vernetzungsreaktion gebracht. Nach der Bestrahlung zeigte die Haftkleberschicht eine erheblich ver-besserte Kohäsion und war praktisch nicht mehr fließfähig, wie aus Fig.3 ersichtlich.

### Beispiel 8

### Untersuchung der Eigenschaften eines mit einem erfindungsgemäß vernetzten Haftkleber beschichteten Substrates:

100 g unvernetzter Haftschmelzkleber der Zusammensetzung 70 Gew.% 2-Ethylhexylacrlat, 10 Gew.% Acrylsäure, 5 Gew.% Methylacrylat, 5 Gew.% 2-Hydroxyethylacrylat, 3 Gew.% Acrylamid, 4 Gew.% N,N'-Trichloracryloyl - 2,6,-diaminotoluol und 3 Gew.% Irgacure 184 wurden im geschmolzenen Zustand bei einer Verarbeitungstemperatur von 120^{o}C auf eine Polyesterfolie, die hier als Substrat eingesetzt wurde, aufgetragen und durch Bestrahlung mit einer UV-Lampe vernetzt. Die Eigenschaften des derart auf dem Substrat hergestellten Haftklebers wurden unter folgenden Bedingungen ermittelt (nach AFERA 4001, 4012):

| | |
|---|---|
| Klebstoffschichtdicke : | 0,1 mm |
| Vernetzung: | Bestrahlung mit UV-Lampe UVRC 500 Leistung: 500 W |
| Bestrahlungszeit: | 1 - 60 sec. |
| Bestrahlungsabstand: | 40 mm |
| Belastung | 30 N |

In Fig. 1 der begleitenden Zeichnung ist die Haltedauer der Klebung in Abhängigkeit von der Bestrahlungszeit bei einem Kleberauftrag von 0,25 mm dargestellt. Hier ist zu beobachten, daß bis zu einer Bestrahlungzeit von 60 sec. die Haltedauer stetig steigt, danach aber eine Abreaktion des Vernetzers stattgefunden hat, sodaß weitere Bestrahlung keinen Effekt mehr zeigt.

### Herstellung eines transdermalen therapeutischen Systems

100 g Haftschmelzkleber der allgemeinen Zusammensetzung 70 Gew.% Butylacrylat, 20 Gew.% Dimethylaminoethylmethacrylat, 7 Gew.% Acrylsäure und 3 Gew.% N,N'-Trichlor-acryloyl-2-6-diaminotoluol wurden geschmolzen und auf 100^{o}C mit 10 g Bopindololhydrogenmalonat vermischt. Die erhaltene Mischung wurde sodann in einer Schichtdicke von 200 g/m² auf aluminiumbedampfte Polyesterfolie aufgebracht. Dieses Zwischenprodukt mit einer sehr viskosen Haftkleberschicht mit nicht ausreichender Kohäsion wurde unter einer Bestrahlungsstrecke mit einer UV-Lampe von 500 W in einem Abstand von 10 cm durchgeführt. Es wurde eine Haftkleberschicht mit stark verbesserter Ko- und Adhäsion erhalten.

## Patentansprüche

1. Haftschmelzkleber, dadurch gekennzeichnet, daß der Haftschmelzkleber aus einer strahlungsnachvernetzbaren Haftschmelzkleberzusammensetzung hergestellt ist.

2. Haftschmelzkleber nach Anspruch 1, dadurch gekennzeichnet, daß der Haftschmelzkleber auf der Basis von Styrol-Isopren-Styrol-Blockpolymeren, Polyisobuten, Ethylen-Vinylacetat-Copolymeren, Polyvinylethern, Acrylaten hergestellt ist und handelsübliche strahlungsinitiierte Vernetzer sowie ggf. Radikalbildner aufweist.

3. Haftschmelzkleber nach Anspruch 1, dadurch gekennzeichnet, daß er aus einer Haftschmelzkleberzusammensetzung auf Acrylatbasis mit einen Gehalt von etwa 0,01 bis 15,00 Gew.%, bevorzugt 0,1 bis 10 Gew.% einer Verbindung der allgemeinen Formel: 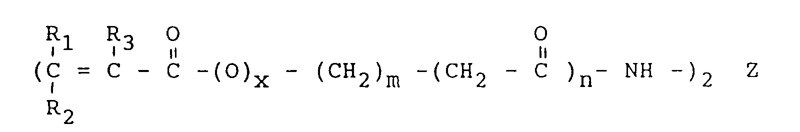 wobei R₁, R₂ = H oder F, Cl, Br oder J ; x = 0 oder 1; n = 0,1; Z = 2,4-Toluylrest, 2,6-Toluylrest oder 1,5-(1-Methylen-1,3,3-trimethyl)-cyclohexylenrest; R₃ = H, CN, Halogen oder CH₃; m = 0 - 12 sein können, herstellbar ist.

4. Haftschmelzkleber nach einem der vorangehenden Ansprüche, dadurch gekennzeichnet, daß die Haftschmelzklebermischung zusätzlich ggf. Weichmacher, Tackifier, Füllstoffe, Alterungsschutzmittel aufweist.

5. Haftschmelzkleber nach irgendeinem der vorangehenden Ansprüche, dadurch gekennzeichnet, daß der Kleber auf Acrylatbasis ausgewählt ist aus der Gruppe bestehend aus solchen auf Basis von: Methylacrylat, Ethylacrylat, Butylacrylat, Hexylacrylat, Heptylacrylat, 2-Ethylhexylacrylat, 2-Methylheptylacrylat, Isooctylacrylat, n-Nonylacrylat, Iso-nonylacrylat, Decylacrylat und/oder Dodecyl(meth)acrylat.

6. Verfahren zur Herstellung des Haftschmelzklebers nach einem der vorangehenden Ansprüche, dadurch gekennzeichnet, daß man eine Mischung zwischen den Haftschmelzkleberkomponenten und dem strahlungsinitiierbaren Vernetzer herstellt, den Haftschmelzkleber im geschmolzenen Zustand auf Verarbeitunqstemperatur auf ein Substrat aufbringt und den strahlungsvernetzbaren Vernetzer durch Bestrahlung zur Vernetzungsreaktion bringt.

7. Verwendung des Haftschmelzklebers nach irgendeinem der vorangehenden Ansprüche für Pflaster, transdermale therapeutische Systeme, Klebebänder, Etiketten, Zierleisten u. dgl.
